# EUROPEAN PATENT SPECIFICATION

(11) **EP 4 656 728 B1**
(45) Date of publication and mention of the grant of the patent: **22.07.2026**
(21) Application number: 25173590.8
(22) Date of filing: 30.04.2025
(51) Int. Cl.: A61P 1/04, C12N 15/86, C07K 14/47, A61K 35/28, C12N 5/0775

(54) **METHOD FOR DELAYING SENESCENCE OF HUMAN MESENCHYMAL STEM CELLS AND APPLICATION THEREOF**
VERFAHREN ZUR VERZÖGERUNG DER SENESZENZ MENSCHLICHER MESENCHYMALER STAMMZELLEN UND ANWENDUNG DAVON
PROCÉDÉ POUR RETARDER LA SÉNESCENCE DE CELLULES SOUCHES MÉSENCHYMATEUSES HUMAINES ET APPLICATION ASSOCIÉE

(30) Priority: 30.05.2024 CN 202410688374
(43) Date of publication of application: 03.12.2025
(73) Proprietor: Affiliated Hospital of Zunyi Medical University, Zunyi Guizhou (CN)
(72) Inventor: XIAO, Jianhui, Zunyi, Guizhou (CN); ZHU, Xinxin, Zunyi, Guizhou (CN); LUO, Yi, Zunyi, Guizhou (CN)
(74) Representative: Murgitroyd & Company

(56) References cited:
- CN-A- 115 029 307
- CN-A- 117 925 840
- CN-B- 112 941 106
- PATEL BHAUMIK B. ET AL: "Schlafen 3, a novel gene, regulates colonic mucosal growth during aging", AMERICAN JOURNAL OF PHYSIOLOGY - GASTROINTESTINAL AND LIVER PHYSIOLOGY, vol. 296, no. 4, 1 April 2009 (2009-04-01), US, pages G955 - G962, XP093300257, ISSN: 0193-1857, DOI: 10.1152/ajpgi.90726.2008

## Description

### FIELD OF THE INVENTION

The invention relates to the field of stem cells and regenerative medicine, in particular, to a method for delaying senescence of human mesenchymal stem cells using a Schlafen (SLFN) family gene member 11 and an application thereof.

### BACKGROUND OF THE INVENTION

Mesenchymal stem cells (MSC) have excellent functional characteristics such as multiple differentiation potential, immune regulation, and promotion of tissue regeneration and repair, as well as advantages such as low immunogenicity and non-tumorigenicity. They are the most promising cell resource in the field of stem cells and regenerative medicine. In 1995, Lazarus et al. first reported the clinical trial of MSC. Especially after the International Society for Cell & Gene Therapy recommended the homogeneity standard of MSC in 2006, clinical trials of MSC have sprung up all over the world. There are more than 1500 clinical trial projects of MSC registered with the US Clinical Trial Registry for the treatment of various diseases, which can be said to have opened a new era of stem cell therapy. However, compared with the large number of clinical trial projects, there are only 10 MSC products approved for marketing by regulatory agencies, and none of them have yet been approved for marketing by regulatory agencies in China or the United States. The reason is that the inconsistent, unstable and potential risks of the therapeutic effects of MSC products hinder their clinical application. This is largely attributed to the fact that the MSC population prepared by in vitro expansion undergoes cell senescence, which leads to the prominent heterogeneity and loss of function of MSCs. It is well known that the clinical use of MSCs from different sources requires the use of in vitro expansion technology to obtain the cell number or cell biomass that meets the clinical treatment dose. However, it is difficult for in vitro cell expansion to avoid the influence of the cell's own genetic and physiological factors and external environmental factors. Recent studies have shown that the gene expression profile of newly isolated primary MSCs undergoes tremendous changes after expansion and culture. The primary MSCs have multiple functions such as extracellular structural organization, collagen biosynthesis, and vascular development. The expanded MSCs lose their inherent functions due to the "proliferation-differentiation-senescence" process. Moreover, the occurrence of senescence not only causes the progressive loss of excellent biological characteristics of MSCs, such as self-renewal, proliferation ability, differentiation potential, immune regulation, migration and homing ability. The senescent cells also harm surrounding healthy cells by secreting senescence-associated secretory phenotypes, inducing cell reprogramming and causing senescence of healthy cells, thereby forming a vicious cycle. What is more concerning is that the infusion of the senescent MSCs into patients not only has no therapeutic effect, but may even cause risks such as immune rejection and canceration of healthy cells. Therefore, the problem of cell senescence during long-term expansion of MSCs in vitro is the Achilles' heel that affects their clinical application, and is also a common key technical bottleneck that urgently needs to be broken through in the frontier of stem cell and regenerative medicine.

From the perspective of the causes of cell senescence after long-term expansion of MSCs in vitro, there are at least two aspects involved. One is that long-term expansion of MSCs in vitro faces the Hayflick limit, also known as replicative senescence, due to cell aging; the other is that factors such as environmental factors and oxidative stress during the long-term expansion of MSCs in vitro lead to DNA damage and imbalance of the intracellular environment, and eventually the cell senescence occurs as the genetic damage accumulates. (Guo X, Wang J, Zou W, et al. Exploring microenvironment strategies to delay mesenchymal stem cell senescence. Stem Cells Dev 2022; 31: 38-52.). So, how to solve this bottleneck so that in vitro expanded stem cells do not senescent or senescent less frequently while maintaining stem cell functions and inherent biological characteristics? The means adopted by the prior art are as follows.

The first one is to focus on the in vitro culture environment of MSCs. By simulating the in vivo stem cell microenvironment, the environmental conditions of the in vitro cell growth niche can be effectively improved, including culture medium composition, temperature, oxygen content, three-dimensional culture, etc., which can achieve bionic empowerment and can effectively reduce cell senescence caused by environmental stress. (Costa LA, Eiro N, Fraile M, et al. Functional heterogeneity of mesenchymal stem cells from natural niches to culture conditions: implications for further clinical uses. Cell Mol Life Sci 2021; 78: 447-67.; Guo X, Wang J, Zou W, et al. Exploring microenvironment strategies to delay mesenchymal stem cell senescence. Stem Cells Dev 2022; 31: 38-52.; Lee SS , Vu TT, Weiss AS, et al. Stress-induced senescence in mesenchymal stem cells: Triggers, hallmarks, and current rejuvenation approaches. Eur J Cell Biol 2023,102: 151331.).

The second one is a strategy to directly target senescent cells, which timely remove senescent cells or delay cell senescence, reduces the paracrine side effects of senescent cells on normal cells, creates a good growth environment for cell proliferation and achieves the goal of obtaining young daughter cells, and includes specific labeling of senescent cells, timely capture and removal of senescent cells using microfluidic technology. (Chen Z, Jiang K, Zou Z, et al. High-throughput and label-free isolation of senescent murine mesenchymal stem cells. Biomicrofluidics 2020, 14, 034106.), natural compounds targeting to remove senescent cells (Wong PF, Dharmani M, Ramasamy TS. Senotherapeutics for mesenchymal stem cell senescence and rejuvenation. Drug Discov Today 2023, 28:103424.), or delaying cell senescence (Yuan H, Xu Y, Luo Y, et al. Ganoderic acid D prevents oxidative stress-induced senescence by targeting 14-3-3ε to activate CaM/CaMKII/NRF2 signaling pathway in mesenchymal stem cells. Aging Cell 2022; 21: e13686.; Yu C, Yuan H, Xu Y, et al. Hyaluronan delays human amniotic epithelial stem cell senescence by regulating CD44 isoform switch to activate AKT/mTOR signals. Biomed Pharmacother 2024,170:116100), and targeted clearance by immune cells (XIAO Jianhui, LUO Yi, ZHONG Jianjiang, YU Changyi. METHOD AND APPLICATION FOR RESISTING AGING AND ENHANCING STEM CHARACTERISTICS OF HUMAN MESENCHYMAL STEM CELLS, patent No.: ZL 2020108465981; XIAO Jianhui, LUO Yi, ZHONG Jianjiang, YU Changyi. METHOD FOR RESISTING AGING AND ENHANCING STEM CHARACTERISTICS OF HUMAN MESENCHYMAL STEM CELLS, patent No.: EP3957718).

The third one is to transform cells through molecular genetic manipulation to make them highly express telomerase or other anti-senescence related genes (Piper SL, Wang M, Yamamoto A, et al. Inducible immortality in hTERT-human mesenchymal stem cells. J Orthop Res. 2012;30(12):1879-85.; Bodnar AG, Ouellette M, Frolkis M, et al. Extension of life-span by introduction of telomerase into normal human cells. Science. 1998; 279(5349):349-352.; He Y, Ji Q, Wu Z, et al. 4E-BP1 counteracts human mesenchymal stem cell senescence via maintaining mitochondrial homeostasis. Protein Cell 2023, 14: 202-216.).

Schlafen (SLFN) family genes are found only in mammals and encode proteins with high sequence homology. However, each SLFN has functional differences and differential expression in different tissues and species, and is widely expressed in cancer and normal cells. SLFN family members are involved in various cell biological processes in specific tissues, including DNA replication, proliferation, induction of immune response and inhibition of viral replication, as well as enhancing the sensitivity of DNA-targeted anticancer drugs. ( Mavrommatis E, Fish EN, Platanias LC. The schlafen family of proteins and their regulation by interferons. J Interferon Cytokine Res. 2013;33(4):206-10.; Jo U, Pommier Y. Structural, molecular, and functional insights into Schlafen proteins. Exp Mol Med. 2022; 54(6):730-738.). The human SLFN family consists of five genes (*SLFN* 5, 11, 12, 13, and 14) clustered on chromosome 17. SLFN11 was discovered by bioinformatics analysis on the cancer cell database and has two main domains, which are an N-terminal nuclease domain and a C-terminal helicase domain. (Murai J, Thomas A, Miettinen M, et al. Schlafen 11 (SLFN11), a restriction factor for replicative stress induced by DNA-targeting anti-cancer therapies. Pharmacol Ther. 2019; 201: 94-102). SLFN11 has been shown to be important for drug sensitivity in a variety of cancer cell lines. (Murai Y, Jo U, Murai J, et al. SLFN11 inactivation induces proteotoxic stress and sensitizes cancer cells to ubiquitin activating enzyme inhibitor TAK-243. Cancer Res. 2021; 81(11): 3067-3078.; Zhou C, Liu C, Liu W, et al. SLFN11 inhibits hepatocellular carcinoma tumorigenesis and metastasis by targeting RPS4X via mTOR pathway. Theranostics. 2020;10(10):4627-4643.; Metzner FJ, Wenzl SJ, Kugler M, et al. Mechanistic understanding of human SLFN11. Nat Commun.2022; 13(1): 5464.). It is a determining factor for the efficacy of first-line anticancer drugs targeting DNA damage, such as platinum derivatives, topoisomerase (TOP) 1 inhibitors, TOP2 inhibitors, and alkylating agents. Moreover, *SLFN11* is used as a biomarker for the diagnosis and treatment of cancer. (Hamada S, Kano S, Murai J, et al. Schlafen family member 11 indicates favorable prognosis of patients with head and neck cancer following platinum-based chemoradiotherapy. Front Oncol. 2023; 12: 978875.; Coleman N, Zhang B, Byers LA, et al. The role of Schlafen 11 (SLFN11) as a predictive biomarker for targeting the DNA damage response. Br J Cancer. 2021; 124(5): 857-859.). However, there have been no reports on the function of *SLFN11* in delaying cell senescence or anti-senescence.

### BRIEF SUMMARY OF THE DISCLOSURE

In order to solve the above problems, an objective of the invention is to provide a method for delaying senescence of human mesenchymal stem cells using Schlafen (SLFN) family gene member 11 and an application thereof. The method can safely and effectively resist the senescence of human mesenchymal stem cells and maintain pluripotency of stem cells. In order to establish a cell line with stable genetic and biological characteristics, the inventors previously performed long-term subculture on human MSC (hMSC) derived from amniotic membranes of different donors, intending to construct an immortalized hMSC cell line. An "immortal" hMSC (named as NA-hMSC) is isolated from amniotic membranes of a certain donor, and does not show a senescence phenotype with stable stem-like characteristics after continuous proliferation for more than 30 generations. By comparing and analyzing with the whole gene transcriptome of hMSCs derived from amniotic membranes of other donors and verifying through RT-qPCR, several differentially expressed genes are discovered from NA-hMSCs. After the reported cell senescence-related genes are removed, *SLFN11* is found to be a potential candidate gene for hMSC immortality, which is highly expressed in NA-hMSCs and not expressed or extremely low expressed in hMSCs derived from amniotic membranes of other donors. The hMSC lines with relevant gene knockout and overexpression are constructed by lentiviral transfection technology. The NA-hMSC cell line with *SLFN11* gene knocked out quickly shows the senescence phenotype in cells, including up-regulated expression of the senescence marker p16 and an increase in the number of β-galactosidase-positive cells. After the *SLFN11* gene is complemented or the *SLFN11* gene is overexpressed in other hMSCs, the cell senescence phenotype, including the expression level of the senescence marker p16 and the number of β-galactosidase-positive cells, decreases sharply, proving that *SLFN11* is a key gene for maintaining the rejuvenation of NA-hMSCs and a candidate gene for delaying the senescence of human mesenchymal stem cells.

The objective of the invention is realized by the following technical solutions.

A method for delaying senescence of human mesenchymal stem cells, wherein an SLFN11 gene is overexpressed in the human mesenchymal stem cells, and the purpose of delaying the senescence of human mesenchymal stem cells is achieved by overexpressing the SLFN11 gene. The SLFN11 gene of the invention is discovered by comparing the whole genomes of the 30th generation NA-hMSC and the 4th generation hMSC.

Further, the senescence of human mesenchymal stem cells includes a replicative cell senescence and a DNA damage-induced cell senescence.

Further, the above method for delaying senescence of human mesenchymal stem cells specifically includes steps of:
S1, establishing a human mesenchymal stem cell senescence;
S2, using lentivirus to infect the human mesenchymal stem cell senescence model so that the human mesenchymal stem cell senescence model overexpresses the SLFN11.

Further, the human mesenchymal stem cell senescence model comprises a replicative hMSC senescence model and a DNA damage-induced hMSC senescence model.

Further, the replicative hMSC senescence model is obtained by continuously expanding the hMSC in vitro to the 16th generation; the DNA damage-induced hMSC senescence model is obtained by treating the hMSC with camptothecin (CPT, 2 µM) for 2 hours to induce DNA damage to cause senescence.

Moreover, the invention further provides a modified human mesenchymal stem cell, which is obtained through overexpression of the SLFN11 gene in the human mesenchymal stem cells using the above method.

Further, the overexpression of the SLFN11 gene is achieved by infecting the human mesenchymal stem cells with lentivirus.

Preferably, the lentivirus is an overexpressed virus HBLV-h-SLFN11-3xflag-ZsGreen-PURO.

Moreover, the invention further provides an application of the modified human mesenchymal stem cell in the preparation of a drug for treating ulcerative colitis.

Moreover, the invention further provides a drug combination, which includes the above modified human mesenchymal stem cell.

The invention compares the whole genome of the 30th generation (P30) NA-hMSC and the 4th generation (P4) hMSC derived from amniotic membranes of human to find that P30 NA-hMSC highly expresses the SLFN11 gene, while P4 hMSC does not express or expresses the gene very low; through gene knockout technology, knocking out the SLFN11 gene in the 27th generation NA-hMSCs significantly increases the production of β-galactosidase, a senescence marker, and the expression of p16, a senescence-related marker, wherein the stem cells quickly showed the senescence phenotype. It is found that the SLFN11 gene is the key gene to maintain the youthfulness of NA-hMSCs after subculture of P30 generations.

The invention, by adopting the replicative hMSC senescence model and the DNA damage-induced hMSC senescence model and using lentiviral infection technology to make these two senescence cell models overexpress SLFN11, significantly reduces the senescence phenotype of the replicative hMSC aging model and the camptothecin (CPT)-induced DNA damage-induced hMSC senescence model (i.e., reducing DNA damage), reduces the expression of senescence-related factors such as p16 and p21, reduces the proportion of β-galactosidase-positive cells, increases the expression levels of cyclin-dependent kinases CDK1 and CDK2, reverses cell cycle arrest, increases the proportion of EdU-positive cells, improves the proliferation capacity of the senescence hMSC model, and also promotes the expression of stem-like transcription factors such as Nanog, Sox2, Oct4, etc. to maintain the pluripotency of hMSC. The SLFN11 gene can promote the expression of Bcl-2 anti-apoptotic protein, reduce the expression of pro-apoptotic enzymes and proteins such as Caspase-3 and Bax, improve the anti-apoptotic ability of hMSCs, and reduce cell apoptosis. The hMSCs overexpressing the SLFN11 gene constructed by lentiviral infection has normal chromosomal karyotype and are inoculated subcutaneously into a nude mouse, then it is shown that there is no chromosomal abnormalities or tumorigenicity. The cells are also used for transplantation into a mouse model with ulcerative colitis, then it is shown that there is no immune exclusion reaction and has better therapeutic effects than normal hMSCs.

In summary, the method of the invention can safely and effectively resist the senescence of human mesenchymal stem cells (hMSCs) and maintain the pluripotency of stem cells.

### Brief Description of the Drawings

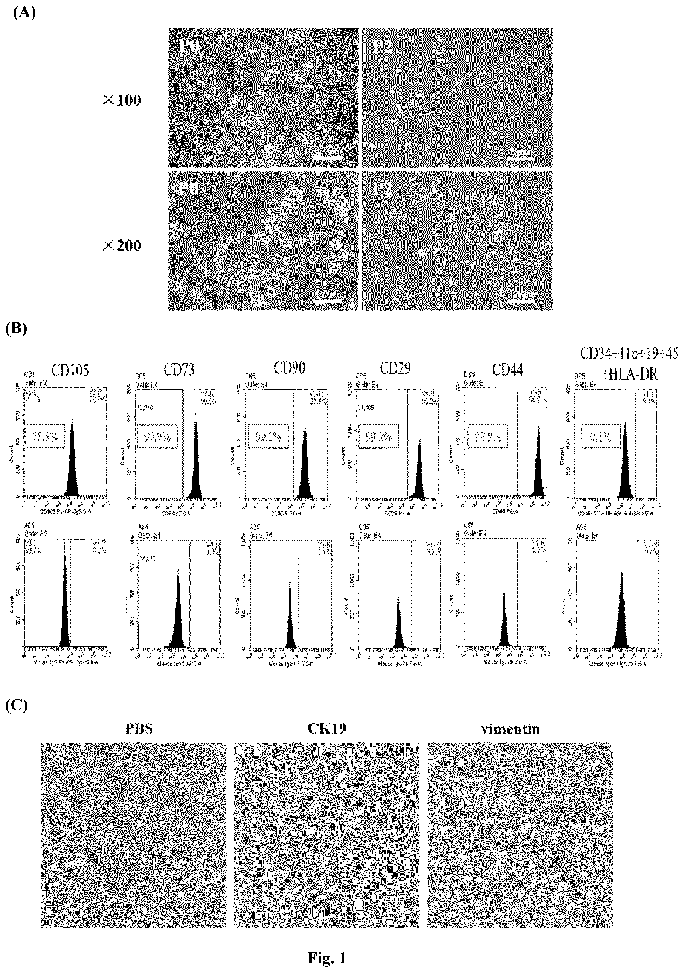

Fig. 1 is a diagram showing typical phenotypic characteristics of human mesenchymal stem cells (hMSCs), wherein (A) is a diagram of cell morphological characteristics; (B) is the detection of surface molecular markers by flow cytometry; (C) is detection of vimentin and keratin CK19 by immunocytochemical staining.

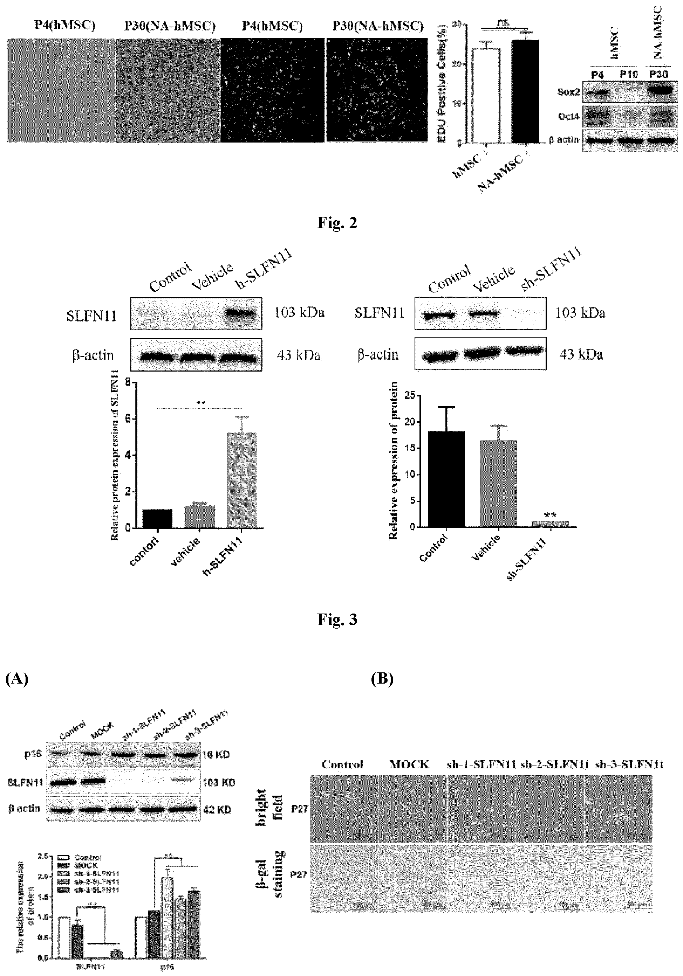

Fig. 2 a diagram showing a comparison of stem cell biological characteristics such as cell morphologies, proliferation capabilities and stem-like factors of P4 generation hMSC and P30 generation NA-hMSC.
Fig. 3 a diagram showing a comparison of the effects of overexpression of the *SLFN11* gene with hMSC and knockout of the *SLFN11* gene with NA-hMSC on the expression the SLFN11 protein.
Fig. 4 is a diagram showing a comparison of the accelerated senescence of NA-hMSCs by knocking out the *SLFN11* gene, wherein (A) is quantitative analysis on the expression of the p21 protein by Western blot; (B) is the β-galactosidase staining.

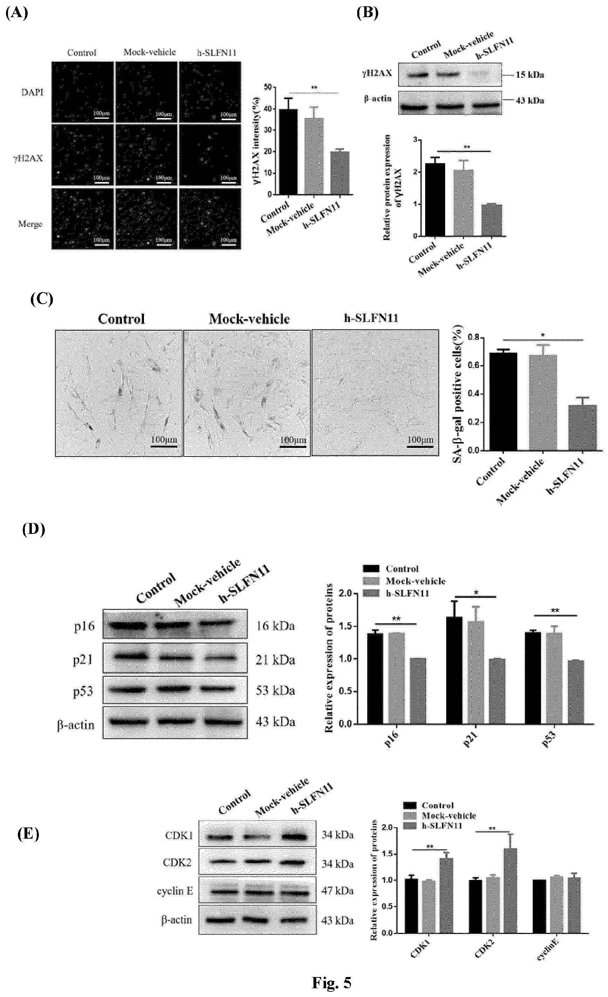

Fig. 5 is a diagram showing a comparison of the effects of the overexpresseion of the *SLFN11* gene on the senescence of long-term in vitro expansion of replicative cells in hMSCs, wherein (A) is the detection of DNA damage marker γH2AX by immunofluorescence staining; (B) is quantitative analysis on DNA damage marker γH2AX by Western blot; (C) is the β-galactosidase staining; (D) is quantitative analysis on the expression of p16 and p21; (E) is the quantitative analysis of the expression on cyclin-dependent kinases CDK1 and CDK2 by Western blot.

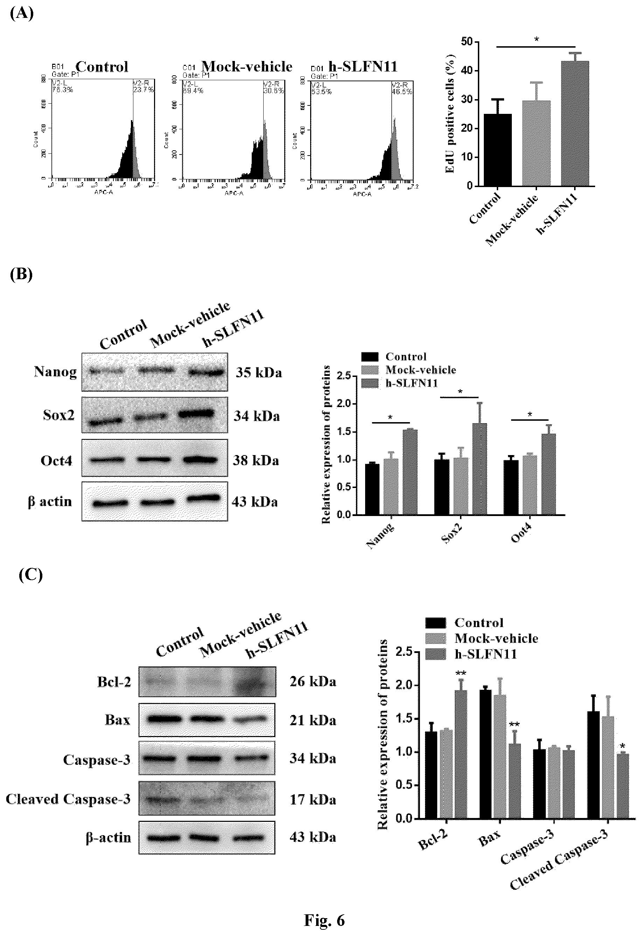

Fig. 6 is a diagram showing a comparison of the improvements of proliferation capacities, pluripotencies and anti-apoptosis capabilities of long-term in vitro expansion of hMSC by overexpression of *SLFN11,* wherein (A) is the detection of cell proliferation capabilities by EdU; (B) is quantitative analysis on the expression level of stem-like transcription factors by Western blot; (C) is quantitative analysis on the expression of apoptosis-related proteins by Western blot.

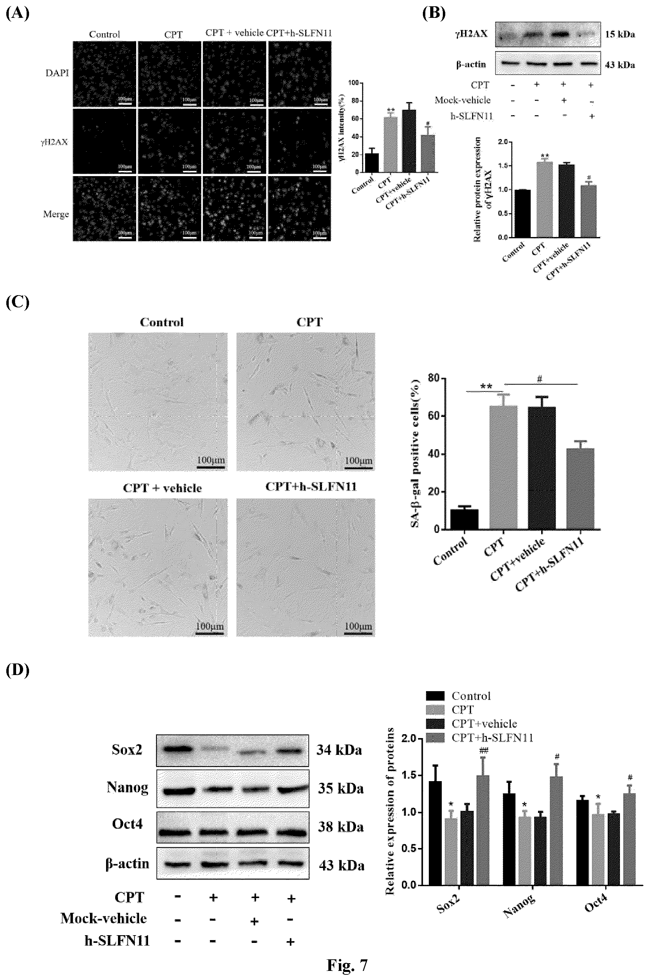

Fig. 7 is a diagram showing a comparison of the effects of the overexpression of the *SLFN11* gene on the senescence of camptothecin-induced hMSC, wherein (A) is the detection of DNA damage marker γH2AX by immunofluorescence staining; (B) is quantitative analysis on DNA damage marker γH2AX by Western blot; (C) is the β-galactosidase staining; (D) is the quantitative analysis on stem-like transcription factors by Western blot.

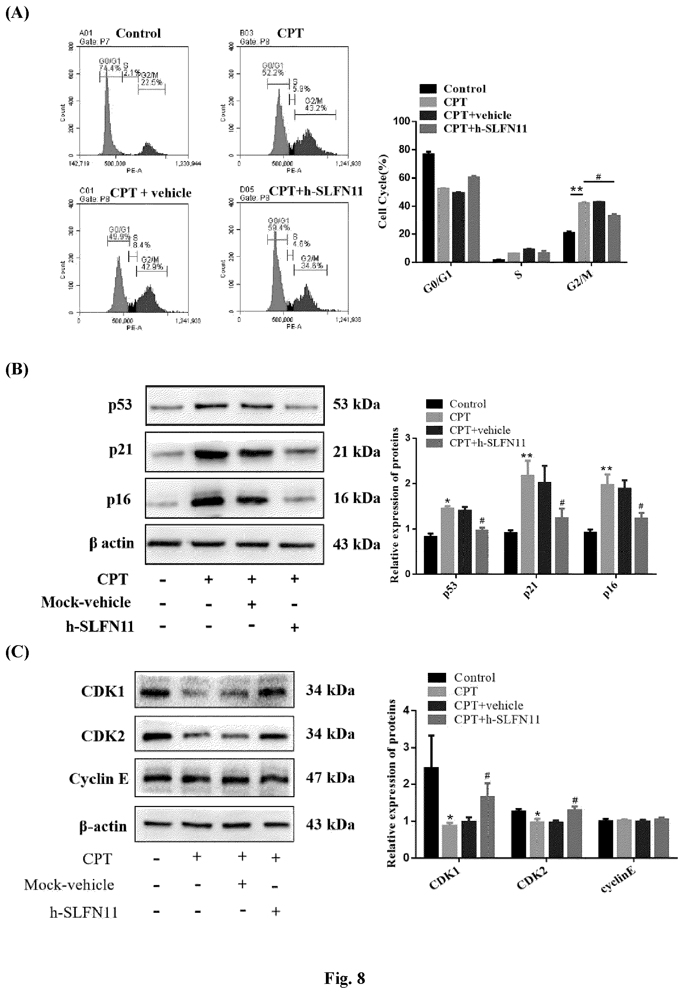

Fig. 8 is a diagram showing a comparison of the effects of the overexpression of the *SLFN11* gene on the cell cycle of the senescence of camptothecin-induced hMSC, wherein (A) is the detection of the cell cycle by flow cytometry; (B) is quantitative analysis on the expression of p16 and p21 by Western blot; (C) is quantitative analysis on the expression of cyclin-dependent kinases CDK1 and CDK2 by Western blot.

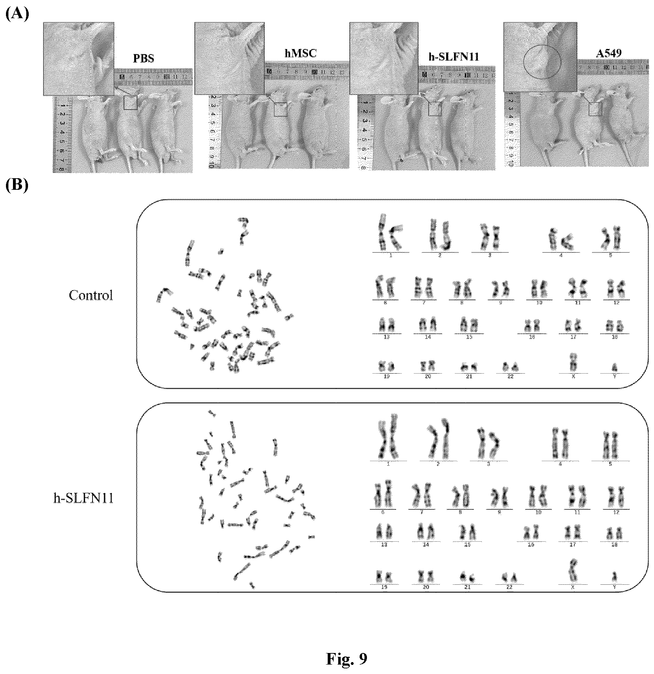

Fig. 9 is a diagram showing a comparison of the safety of hMSC overexpressing the *SLFN11* gene; wherein (A) is the tumorigenicity test; (B) is the analysis on chromosome karyotype.

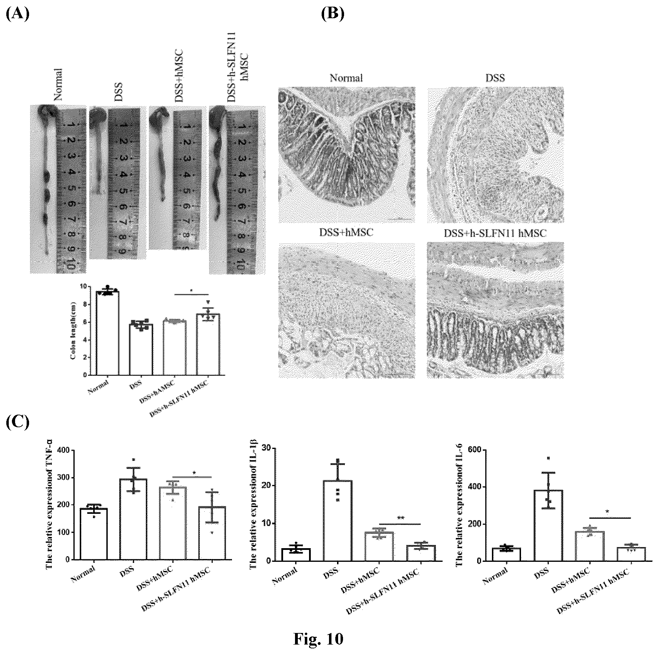

Fig. 10 is a diagram showing a comparison of the efficacy and evaluation of transplantation of hMSC overexpressing the *SLFN11* gene in the treatment of mice with ulcerative colitis, wherein (A) shows changes in colon length of the mice; (B) is pathological analysis on colon tissues by H&E staining; (C) is the detection of the expression of inflammatory factors by ELISA.

### Detailed Description of the Invention

Through specific examples of the present invention below, those skilled in the art can easily understand other advantages and efficacies of the present invention revealed by the specification. The described embodiments are only a part of the preferred embodiments of the present invention, but not the whole. Based on the embodiments of the invention, all other embodiments obtained by a person skilled in the art without involving any inventive effort are within the scope of the invention.

### Embodiment 1: Isolation, culture and identification of human mesenchymal stem cells (hMSC)

The instruments and PBS buffer (phosphate buffered saline) required for amniotic membrane extraction are sterilized by high pressure. The placenta after cesarean section is moved to a sterile square dish, and the blood stains on the placenta surface are washed with PBS buffer containing 1% PS. Scissors and forceps are used to peel off the amniotic membrane tissue on the placenta to be placed in a wide-mouth bottle filled with PBS buffer.
In a clean environment such as a clean bench, the amniotic membrane is cut into pieces (about 2-5 cm²), which are transferred into a 50 mL centrifuge tube; then 0.2% EDTA-2Na-0.5% trypsin is added; after the centrifuge tube is sealed with a sealing film, the centrifuge tube is shaken and digested in a constant temperature shaker at 37±5°C for 90 minutes, and filtered with a 300-mesh filter to remove the filtrate. The amniotic membrane after 3 filtrations is washed and transferred into a 50 mL centrifuge tube, and then 0.5 mg/mL type II collagenase-0.05 mg/mL DNasel digestion solution at a volume ratio of 1:1 to amniotic membrane is added; the centrifuge tube is shaken and digested in a constant temperature shaker at 37±5°C for about 40-60 minutes until the amniotic tissue becomes flocculent. The centrifuge tube is filtered with a 300-mesh filter into a 50 mL centrifuge tube and centrifuged at 250 × g for 10 minutes to discard the supernatant for precipitating; then, an appropriate amount of complete culture medium is added to resuspend by pipetting, and inoculated in a T75 culture flask for culturing in a 37°C, 5% CO₂ incubator, wherein the adherent cells are primary hMSCs.

After subculture and purification of the primary hMSCs, the second generation (P2) hMSCs show typical fibroblastic shape and grow in a whirlpool shape with a distinct three-dimensional sense (as shown in image A in Fig. 1).

The results of flow cytometry show (as shown in image B of Fig. 1) that the hMSC highly expresses MSC surface molecules CD105 (78.8%), CD73 (99.9%), CD90 (99.5%), CD44 (99.2%) and CD29 (98.9%), and negatively expressed hematopoietic stem cell markers CD34+11b+19+45+HLA-DR (0.1%). The results of immunocytochemical staining show (as shown in image C of Fig. 1) that the hMSC highly expresses the MSC surface marker vimentin, but does not express the epithelial cell marker keratin CK19. The isolated and prepared hMSCs have typical MSC characteristics and high purity.

By comparing the relevant stem cell biological characteristics of P4 (4th generation) hMSCs and P30 (30th generation) NA-hMSCs, it is observed that the cell morphology of P30 generation NA-hMSCs is like that of young P4 generation hMSCs, showing fibroblastic and swirling growth (as shown in right bright field image in Fig. 2 ); the number of EdU-positive cells is slightly higher than that of hMSCs at generation P4, which maintain a good proliferation capacity (as shown in the middle immunofluorescence image in Fig. 2); the protein immunoblot on the left in Fig. 2 shows that when P4 hMSCs are subcultured to P10, the expression levels of stem-like factors such as Oct4 and Sox2 are sharply downregulated, while the expression levels of stem-like factors such as Oct4 and Sox2 in P30 NA-hMSCs are not lower than those in P4 hMSCs and are significantly stronger than those in P10 hMSCs. Therefore, P30 NA-hMSCs still maintain the biological characteristics of young hMSCs, such as morphological characteristics, proliferation capabilities and multipotencies.

### Embodiment 2: Construction of hMSCs stably overexpressing/knockout SLFN11 by recombinant lentiviral infection

The *SLFN11* overexpression/knockout lentivirus is constructed by Shanghai Hanheng Biotechnology Co., Ltd. The cloning vector was pHBLV, and the nucleotide sequence of the *SLFN11* gene is shown in SEQ ID NO.1.

SEQ ID NO .1:

atggag gcaaatcagt gccccctggt tgtggaacca tcttacccag acctggtcat caatgtagga gaagtgactc

ttggagaaga aaacagaaaa aagctgcaga aaattcagag agaccaagag aaggagagag ttatgcgggc tgcatgtgct

ttattaaact caggaggagg agtgattcga atggccaaga aggttgagca tcccgtggag atgggactgg atttagaaca gtctttgaga

gagcttattc agtcttcaga tctgcaggct ttctttgaga ccaagcaaca aggaaggtgt ttttacattt ttgttaaatc ttggagcagt

ggccctttcc ctgaagatcg ctctgtcaag ccccgccttt gcagcctcag ttcttcatta taccgtagat ctgagacctc tgtgcgttcc

atggactcaa gagaggcatt ctgtttcctg aagaccaaaa ggaagccaaa aatcttggaa gaaggacctt ttcacaaaat

tcacaagggt gtataccaag agctccctaa ctcggatcct gctgacccaa actcggatcc tgctgaccta attttccaaa aagactatct

tgaatatggt gaaatcctgc cttttcctga gtctcagtta gtagagttta aacagttctc tacaaaacac ttccaagaat atgtaaaaag

gacaattcca gaatacgtcc ctgcatttgc aaacactgga ggaggctatc tttttattgg agtggatgat aagagtaggg aagtcctggg

atgtgcaaaa gaaaatgttg accctgactc tttgagaagg aaaatagaac aagccatata caaactacct tgtgttcatt tttgccaacc

ccaacgcccg ataaccttca cactcaaaat tgtgaatgtg ttaaaaaggg gagagctcta tggctatgct tgcatgatca gagtaaatcc

cttctgctgt gcagtgttct cagaagctcc caattcatgg atagtggagg acaagtacgt ctgcagcctg acaaccgaga aatgggtagg

catgatgaca gacacagatc cagatcttct acagttgtct gaagattttg aatgtcagct gagtctatct agtgggcctc cccttagcag

accagtgtac tccaagaaag gcctggaaca taaaaaggaa ctccagcaac ttttattttc agtcccacca ggatatttgc gatatactcc

agagtcactc tggagggacc tgatctcaga gcacagagga ctagaggagt taataaataa gcaaatgcaa cctttctttc ggggaatttt
gatcttctct agaagttggg ctgtggacct gaacttgcag gagaagccag gagtcatctg tgatgctctg ctgatagcac agaacagcac
ccccattctc tacaccattc tcagggagca agatgcagag ggccaggact actgcactcg caccgccttt actttgaagc agaagctagt
gaacatgggg ggctacaccg ggaaggtgtg tgtcagggcc aaggtcctct gcctgagtcc tgagagcagc gcagaggcct
tggaggctgc agtgtctccg atggattacc ctgcgtccta tagccttgca ggcacccagc acatggaagc cctgctgcag tccctcgtga
ttgtcttact cggcttcagg tctctcttga gtgaccagct cggctgtgag gttttaaatc tgctcacagc ccagcagtat gagatattct
ccagaagcct ccgcaagaac agagagttgt ttgtccacgg cttacctggc tcagggaaga ccatcatggc catgaagatc
atggagaaga tcaggaatgt gtttcactgt gaggcacaca gaattctcta cgtttgtgaa aaccagcctc tgaggaactt tatcagtgat
agaaatatct gccgagcaga gacccggaaa actttcctaa gagaaaactt tgaacacatt caacacatcg tcattgacga
agctcagaat ttccgtactg aagatgggga ctggtatggg aaggcaaaaa gcatcactcg gagagcaaag ggtggcccag
gaattctctg gatctttctg gattactttc agaccagcca cttggattgc agtggcctcc ctcctctctc agaccaatat ccaagagaag
agctcaccag aatagttcgc aatgcagatc caatagccaa gtacttacaa aaagaaatgc aagtaattag aagtaatcct tcatttaaca
tccccactgg gtgcctcgag gtatttcctg aagccgaatg gtcccagggt gttcagggaa ccttacgaat taagaaatac ttgactgtgg
agcaaataat gacctgtgtg gcagacacgt gcaggcgctt ctttgatagg ggctattctc caaaggatgt tgctgtgctt gtcagcaccg
caaaagaagt ggagcactat aagtatgagc tcttgaaagc aatgaggaag aaaagggtgg tgcagctcag tgatgcatgt
gatatgttgg gtgatcacat tgtgttggac agtgttcggc gattctcagg cctggaaagg agcatagtgt ttgggatcca tccaaggaca
gctgacccag ctatcttacc caatgttctg atctgtctgg cttccagggc aaaacaacac ctgtatattt ttccgtgggg tggccattag。

P3 (the 3rd generation) hMSC and P30 (the 30th generation) NA-hMSC are inoculated in 6-well plates, and the virus stock solution is added according to the 1/2 volume infection method. The P3 hMSCs are divided into a negative control group (Control, without virus solution), a mock-vehicle group (Mock-vehicle, abbreviated as "vehicle" in the left image of Fig. 3, with mock-vehicle virus HBLV-ZsGreen-PURO added) and an SLFN11 overexpression group (h-SLFN11, with overexpressed virus HBLV-h-SLFN11-3xflag-ZsGreen-PURO added). The P30 NA-hMSCs are divided into a negative control group (Control, without virus solution), a mock-vehicle control group (Mock-vehicle, abbreviated as "vehicle" in the right image of Fig. 3, with mock-vehicle virus HBLV-ZsGreen-PURO added) and an SLFN11 knockout group (sh-SLFN11, with knockout virus HBLV-h-SLFN11- shRNA-mcherry-PURO added). After 24 hours of exposure, the culture medium of each group is replaced with fresh complete culture medium. Western blot is used to detect the transfection effect. The results are shown in Fig. 3 (the detection image of P3 generation hMSC is shown in the left image of Fig. 3, and the detection image of P30 NA-hMSC is shown in the right image of Fig. 3). After 72 hours of infection, the hMSCs that do not express or weakly express SLFN11 protein are observed. After the *SLFN11* gene is overexpressed (the h-SLFN11 group), the expression level of SLFN11 protein increases sharply, while after knocking out the *SLFN11* gene (the sh-SLFN11 group) in NA-hMSCs that highly express the SLFN11 protein, the expression level of the SLFN11 protein decreases sharply, showing that the lentiviral infection is effective.

### Embodiment 3: Establishment of cell senescence model

Construction of the replicative MSC senescence model: the hMSCs are cultured in vitro for a long time until P16 generation, and the P16 hMSCs have a typical senescence phenotype, with cell senescence markers such as β-galactosidase, p16, and p21 detected (see image C in Fig. 5 and image D in Fig. 5 for the Control group for details), which can be used as the replicative senescence model.

Construction of the CPT-induced DNA damage-induced MSC senescence model (DNA damage-induced hMSC senescence model): the hMSCs of the P3 generation with good growth conditions are taken and treated with CPT at a final concentration of 2 µM for 2 hours, and then replaced with fresh complete culture medium; after 48 hours of culture, the cells have a typical senescent phenotype, and cell senescence markers such as β-galactosidase, p16, and p21 are detected (see the CPT group in image C in Fig. 7 and the CPT group in image B in Fig. 8 for details).

### Embodiment 4: Effects of SLFN11 knockout on senescence of NA-hMSCs

The NA-hMSCs of the 24th generation are taken and inoculated into a 6-well cell culture plate at a density of 3×10⁵/well; the virus stock solution is added after culturing for 24 hours, and the culture medium is replaced with fresh culture medium after 24 hours; then, the subculture is continued to the 27th generation (P27). The P27 NA-hMSCs are taken and divided into a negative control group (Control, without virus solution), an mock-vehicle negative control group (Mock-vehicle, abbreviated as Mock in Fig. 4, with mock-vehicle virus HBLV-ZsGreen-PURO added) and three SLFN11 knockout groups (sh-1-SLFN11, sh-2-SLFN11 and sh-3-SLFN11, with knockout virus HBLV-h-SLFN11- shRNA-mcherry-PURO added). The above groups are inoculated into a 12-well and a 6-well cell culture plate at a density of 2×10⁵/well and 5×10⁵/well, respectively. After culturing for 48 hours, the production of β-galactosidase in each group is detected, and the total cell protein is extracted to detect the expression of senescence-related protein p16. As shown in Fig. 4, the NA-hMSCs with *SLFN11* knockout (the SLFN11 knockout group) significantly increase the number of β-galactosidase-positive cells (blue), and the expression level of p16 is significantly upregulated (p<0.01). Therefore, the *SLFN11* knockout may cause the immortal cell line NA-hMSC to lose the anti-senescence capability.

### Embodiment 5: Effects of SLFN11 on replicative senescence of long-term in vitro expansion of hMSCs

The hMSCs are continuously expanded in vitro to the P16 generation; the hMSCs are divided into a negative control group (Control, without virus solution), an mock-vehicle negative control group (Mock-vehicle, with mock-vehicle virus HBLV-ZsGreen-PURO added) and an SLFN11 overexpression group (h-SLFN11, with overexpression virus HBLV-h-SLFN11-3xflag-ZsGreen-PURO added). The cells of each group are grown to 80%-90% in the plate, fixed with 4% PFA for 30 min, washed three times with PBS, permeabilized with 0.3% Triton X-100 for 10 min, washed three times with PBS, blocked with 0.5% BSA for 1 h, and finally incubated with a primary antibody at 4°C overnight. The next day, the cells are incubated with a fluorescent secondary antibody from the same source in the dark for 1 h and observed under a microscope. The results of immunofluorescence staining of DNA damage marker yH2AX (as shown in image A in Fig. 5) suggest that the fluorescence intensity and number in the nucleus of the control group are significantly higher than those of hMSCs overexpressing *SLFN11* (the h-SLFN11 group), indicating that overexpression of *SLFN11* may effectively alleviate the accumulation of DNA damage during long-term in vitro expansion of hMSCs. Further, the yH2AX protein immunoblot quantitative analysis obtains consistent results. The overexpression of *SLFN11* may significantly reduce the expression level of yH2AX protein (*p*<0.01)(as shown in image B in Fig. 5). Therefore, the overexpression of *SLFN11* may effectively reduce the accumulation of DNA damage caused by replicative senescence of hMSCs.

The β-galactosidase positive cell rate of hMSCs at the P16 generation is detected by β-galactosidase staining. The β-galactosidase staining working solution is prepared according to the kit, the cells are taken out at the corresponding time point, the culture medium in the well s aspirated, and then 1 mL of fixative is added for fixation at room temperature for 30 min, followed by washing 3 times with PBS, adding 1 mL of the staining working solution to each well and incubating in a 37°C water bath until obvious blue-stained cells appear; finally, the observation is performed under an optical microscope. As shown in image C in Fig. 5, after long-term in vitro expansion culture, the intracellular β-galactosidase accumulates in hMSCs and the number of positive cells (blue-stained cells) increase; however, in hMSCs overexpressing the *SLFN11* gene (the h-SLFN11 group), the cell positive rate decreases from (74.52±2.15)% to (37.28±4.73)%. Therefore, the overexpression of *SLFN11* may effectively reduce the replicative senescence of hMSCs.

Based on the common existence of important cell senescence phenotypes in senescent cells, such as cell cycle arrest, weakened proliferation capability, and progressive loss of stem cell pluripotency, the effect of *SLFN11* on changing these senescence phenotypes of hMSCs is evaluated. The cell proteins of the corresponding groups are extracted and denatured, and the expression levels of related proteins are detected by Western blot. The results are as follows: for the P16 generation cells of hMSCs after long-term passage (Control group), the expression of cell cycle regulatory proteins (also senescence-related markers) p16, p21, and p53 are increased (as shown in image D in Fig.5), and the expression of cyclin-dependent kinase (CDK) 1 and CDK 2 is decreased, which lead to cell cycle arrest (as shown in image E in Fig. 5); while for the P16 generation hMSCs overexpressing *SLFN11,* the expression levels of proteins p16, p21 and p53 (*p*<0.05 or *p*<0.01) (as shown in image D in Fig. 5) are effectively reduced, and the expression levels of proteins CDK1 and CDK2 (*p*<0.05 or *p*<0.01) are increased, which lead to reversal of cell cycle arrest (as shown in image E in Fig. 5). Therefore, the overexpression of *SLFN11* may regulate the expression levels of cell cycle regulatory proteins and cell cycle-dependent protein kinases as well as reversing the cell cycle arrest caused by replicative senescence of hMSCs.

### Embodiment 6: Improvement of overexpression of SLFN11 for proliferation capabilities, pluripotencies and anti-apoptosis capabilities of stem cells

EdU kit is used to detect cell proliferation capabilities; the hMSCs are divided into a negative control group (Control, without virus solution), an mock-vehicle negative control group (Mock-vehicle, with mock-vehicle virus HBLV-ZsGreen-PURO added) and an SLFN11 overexpression group (h-SLFN11, with overexpression virus HBLV-h-SLFN11-3xflag-ZsGreen-PURO added); the cells are cultured in a 6-well plate until the corresponding time point, the original culture medium is aspirated, 1× EdU working solution is added, and then the cells are incubated in an incubator for 2 h. After the cells are labeled with EdU for a certain time, the culture medium is removed, and the cells are digested with trypsin and resuspended in PBS to collect the precipitate by centrifugation; 1 mL of 4% PFA fixative is added for fixation at room temperature for 30 min, and 1 mL of 0.3% TritonX-100 is added to permeabilize the membrane at room temperature for 15 min; the reaction working solution is prepared according to the test instructions, and 0.5 mL of the reaction working solution is added to each tube and incubated at room temperature in the dark for 30 min. Then, PBS is used to wash three times, 3-5 min each time. And then, on-machine testing is performed. From image A in Fig. 6, the results show that when hMSCs are expanded in vitro to P16, the proportion of EdU positive cells is only (24.67±5.28)%, while the proportion of EdU positive cells reaches (47.18±2.63)% in the P16 generation hMSCs overexpressing *SLFN11,* which indicate that the overexpression of *SLFN11* may enhance cell proliferation capabilities. The expression of three main stem-like factors, Nanog, Sox2, and Oct4, also indicates that the expression levels of the three stem-like factors in hMSCs overexpressing the *SLFN11* gene are significantly higher than those in the Control group (*p*<0.05) (as shown in image B in Fig. 6), thereby indicating that the overexpression of *SLFN11* may effectively maintain the multipotency characteristics of hMSCs. In addition, the effects of the overexpression of *SLFN11* on apoptosis in the replicative senescence MSC model are further examined, and the results are shown in image C in Fig. 6, wherein in the *SLFN11* overexpression group, the expression of Bcl-2 is upregulated (*p*<0.01), while the expressions of splicing forms of Bax and Caspase-3 are significantly downregulated (*p*<0.05 or *p*<0.01). In summary, the overexpression of *SLFN11* may reverse hMSC replicative senescence, reshape cell proliferation capabilities, maintain stem cell pluripotencies, enhance cell anti-apoptosis capabilities, and reduce cell apoptosis.

### Embodiment 7: Effects of overexpression of SLFN11 on camptothecin-induced hMSC senescence

In order to further confirm the anti-hMSC senescence function of *SLFN11* gene, a drug-induced hMSC senescence model is constructed. Camptothecin (CPT) can induce DNA damage of cells, leading to cell senescence. P4 generation hMSCs or P4 generation hMSCs overexpressing *SLFN11* are divided into four groups, i.e., a normal control group (Control, without CPT treatment and virus solution), a mock-vehicle negative control group (CPT+vehicle or CPT+Mock-vehicle, treated with CPT and added with mock-vehicle virus HBLV-ZsGreen-PURO) and an overexpression SLFN11 group (CPT+h-SLFN11, treated with CPT and added with overexpression virus HBLV-h-SLFN11-3xflag-ZsGreen-PURO); the cells are inoculated in a 6-well cell culture plate at a density of 1×10⁶/well and treated with CPT (2 µM) for 2 hours. The following indicators are then tested.

After CPT treatment, DNA damage is induced, and the expression of DNA damage marker γH2AX is detected by immunofluorescence staining and WB. As shown in image A in Fig. 7, compared with the normal control group (Control group), the fluorescence intensity (red) and number of γH2AX increase significantly after CPT treatment, while the overexpression of SLFN11 partially alleviated this situation. Further, the expression of γH2AX is detected at the protein level; as shown in image B in Fig. 7, the expression level of γH2AX in the CPT+h-SLFN11 group is significantly decreased compared with that in the CPT group. Therefore, the overexpression of *SLFN11* may effectively alleviate the accumulation of DNA damage manifested by CPT-induced hMSC senescence.

The results of the staining experiment of β-galactosidase, a marker of senescence, show that compared with the normal control group, the number of β-galactosidase positive cells (blue-stained cells) in the CPT group increases significantly, and the positive cell rate increases from (10.83±2.79)% to (65.56±4.37)%, while the positive cell rate of the *SLFN11* overexpression group (CPT+h-SLFN11 group) is only (43.76±3.16)% (as shown in image C in Fig. 7). Therefore, the overexpression of *SLFN11* may effectively reduce CPT-induced hMSC senescence.

In addition, as shown in image D in Fig. 7, compared with the normal control group (Control group), CPT may significantly reduce the expression of Sox2, Nanog, and Oct4, main stem-like factors of hMSCs (*p*<0.05), but may have no effect on hMSCs overexpressing the *SLFN11;* compared with the CPT group, the CPT+h-SLFN11 group significantly enhances the expression levels of these stem-like factors (*p*<0.05 or *p*<0.01). Therefore, the overexpression of *SLFN11* may maintain the stem cell pluripotencies of hMSCs.

### Embodiment 8: Reversal of SLFN11 for cell cycle arrest caused by camptothecin-induced hMSC senescence

Flow cytometry is used to detect the cell cycle, and the cells that grow well are taken and divided into four groups, i.e., a normal control group (Control, without CPT treatment and virus solution), a mock-vehicle negative control group (CPT+vehicle or Mock-vehicle, treated with CPT and added with mock-vehicle virus HBLV-ZsGreen-PURO) and an overexpression SLFN11 group (CPT+h-SLFN11 or h-SLFN11, treated with CPT and added with overexpression virus HBLV-h-SLFN11-3xflag-ZsGreen-PURO); the cells are treated with CPT, then washed once with PBS and centrifuged at 150×g for 5 min to collect the cell pellet. 500 µL of 70% pre-cooled ethanol is added for fixation overnight and storage at 4°C. The fixative is washed away with PBS, followed by adding 100 µL of RNase A solution to the precipitate, mixing carefully by pipetting and incubating in a 37°C water bath for 30 min. 400 µL of PI staining solution is added for mixing well by pipetting, followed by incubating at 4°C in the dark for 30 min. Finally, on-machine detection is performed, and the red fluorescence at the excitation wavelength of 488 nm is recorded. Further, the results are shown in Fig. 8, wherein compared with the normal control group (Control group), CPT treatment causes a significant upregulation of the expression of cell cycle regulatory proteins (also senescence-related markers) p21 (*p*<0.01), p16 (*p*<0.01), and p53 (*p*<0.05) (as shown in image B in Fig. 8), and the expression levels of cell cycle-dependent protein kinases CDK1 and CDK2 are significantly reduced (*p*<0.05) (as shown in Figure 8C), thereby leading to cell cycle arrest at the G2/M phase so that the number of cells in the G2/M phase increases from (20.65±1.31)% to (41.95±0.95)% (as shown in image A in Fig. 8); while for the *SLFN11* group (CPT+h-SLFN11), the proportion of cells in the G2/M phase decreases to (33.07±1.36)% (as shown in image A in Fig. 8), the expression levels of p21, p16, p53 and other proteins are significantly downregulated (p<0.05) (as shown in image B in Fig. 8), and the protein levels of CDK1 and CDK2 are significantly increased (p<0.05) (as shown in image C in Fig. 8). Therefore, the overexpression of *SLFN11* may effectively regulate the expression levels of cell cycle regulatory proteins and cell cycle-dependent protein kinases as well as alleviating the cell cycle arrest caused by CPT-induced hMSC senescence.

### Embodiment 9: Analysis on safety of hMSCs overexpressing SLFN11

After one week of adaptive feeding, 4-week-old BALB/c nude mice are randomly divided into 4 groups, labeled with PBS, hMSC, h-SLFN11 hMSC and A549, with 5 mice in each group, for a total of 20 mice. For the three groups of hMSC, h-SLFN11 hMSC and A549, each nude mouse is injected with 100 µL of normal hMSCs, hMSCs overexpressing *SLFN11,* and non-small cell lung cancer A549 cells (1×10⁷/mouse) under the armpit. The PBS group is injected with 100 µL sterile PBS. During the observation, body weight is measured regularly every week, and tumor growth is observed and recorded. On the 6th day after cell transplantation, obvious masses appear in the A549 group, and the volume of the masses gradually increases with time. On the 35th day after cell transplantation, the A549-bearing mice are killed, the tumors are dissected out, and the length, width and weight are measured. The other groups are continued to be observed until the 90th day. The results of subcutaneous tumor formation experiment for the nude mouse are shown in image A in Fig. 9. Among the solvent control PBS group, the normal hMSC group, the h-SLFN11 hMSC group and the A549 positive control group, only the A549 group nude mice are found to have lumps in the armpits.

After the cells are treated with 40 µg/mL colchicine for 3 hours, the cells are collected and treated with 0.075 mol/L KCl solution for hypotonicity adn the cells are fixed. A pipette is used to draw the cell suspension onto a slide at an appropriate height (10cm-20cm), 2 drops of suspension are added to each slide (the temperature of the slide is required to be 20-25°C, and the humidity is 40%-60%), followed by immediately placing into a 90°C oven for baking. The number of metaphase cells, chromosome length, dispersion and other general morphologies are observed under a phase contrast microscope. The prepared slides are placed in a 37°C oven for baking. The baked chromosome slides are treated with Giemsa working solution for color development, dried and then scanned on machine. The results of analysis on chromosome karyotype are shown in image B in Fig. 9. The chromosome karyotype of hMSCs overexpressing SLFN11 remains normal. In summary, the hMSCs overexpressing SLFN11 constructed by lentiviral infection are non-tumorigenic, have no chromosomal abnormalities, and have no safety issues.

### Embodiment 10: Evaluation on the therapeutic effect of hMSCs overexpressing SLFN11 in the treatment of ulcerative colitis in mice

There are C57BL/6 mice, 7-8 weeks old, male, weighing 20-22g. After one week of adaptive feeding under SPF conditions, the mice are allowed to drink freely 3% DSS-containing drinking water for 7 consecutive days to establish an ulcerative colitis mouse model. A total of 40 mice are randomly divided into 4 groups, 10 mice in each group, and labeled as Normal, DSS, DSS+hMSC, and DSS+h-SLFN11+hMSC respectively. The DSS+hMSC and DSS+h-SLFN11+ hMSC groups are injected with 0.2 mL of normal hMSC and hMSCs overexpressing *SLFN11* (1.0×10⁶/mouse) through the tail vein before and on the fourth day of the establishment of the model, respectively, while the DSS group is injected with 0.2 mL of normal saline. The weight, stool characteristics (loose stool, diarrhea, bloody stool) and mortality of the mice are recorded every day. It is observed that on the fourth and fifth days of DSS feeding, the mice began to show obvious symptoms of inflammatory colitis, including a sharp drop in body weight and bloody stools.

Nine days after treatment with hMSC transplantation, the mice are killed under isoflurane anesthesia, and the intact colon is removed to measured the length. Part of the colon segment is placed in an embedding box, fixed with 4% paraformaldehyde overnight, embedded in paraffin, and made into 4-µm thick histological sections for hematoxylin-eosin (H&E) staining. The crypt structure, inflammatory cell infiltration degree and goblet cell structure of the colon tissue are observed under a microscope. The results are shown in Fig. 10, wherein for the mice in the DSS group, the length of the colon is significantly shortened (as shown in image A in Fig. 10), the morphological structure of the colon tissue is severely damaged, the goblet cells in the mucosal layer are disordered and reduced in number, a large number of inflammatory cells infiltrate, and the crypt structure completely disappears (as shown in image B in Fig. 10); while transplantation of hMSCs may significantly alleviate DSS symptoms, maintain the length of the mouse colon, and reduce pathological changes in colon tissue; in particular, the effect of injection of hMSCs overexpressing *SLFN11* is more obvious, which may better maintain the structural integrity of colon tissue cells (as shown in image B in Fig. 10), and the therapeutic effect is better than that of the hMSC group.

The blood of mice is collected by venous bleeding, the blood is allowed to rest for 2 hours, and the serum is separated by centrifugation. ELISA kits are used to detect the expression of inflammatory factors in serum. The results are shown in image C in Fig. 10, wherein the expression levels of pro-inflammatory factors IL-1β, IL-6, and TNF-α in the serum of mice in the DSS group are significantly increased (p<0.01). Compared with the DSS group, hMSC treatment has a significant inhibitory effect on these inflammatory factors (p<0.01), especially in the hMSC overexpressing *SLFN11* group, which is also significantly different from the hMSC group (p<0.01).

In summary, the hMSCs overexpressing *SLFN11* may significantly alleviate the symptoms of ulcerative colitis in mice and have a better therapeutic effect than normal hMSC transplantation.

Other aspects of the invention that are not described in detail are all conventional techniques known to those skilled in the art.

It should be noted that terms "comprising", "including" or any other variants are intended to cover non-exclusive inclusion, thereby making a process, method, object or apparatus comprising a series of elements comprise not only those elements but also other elements that are not listed explicitly or the elements inherent in the process, method, object or apparatus.

The protection scope of the invention is not limited to the technical solutions disclosed in the specific implementation methods. Any modifications, equivalent substitutions, improvements, etc. made to the above embodiments based on the technical essence of the invention shall fall within the protection scope of the invention.

## Claims

1. A method for delaying senescence of human mesenchymal stem cells, wherein an SLFN11 gene is overexpressed in the human mesenchymal stem cells, and the nucleotide sequence of the SLFN11 gene is shown in SEQ ID NO .1.

2. The method for delaying senescence of human mesenchymal stem cells according to claim 1, wherein the senescence of human mesenchymal stem cells comprises a replicative cell senescence and a DNA damage-induced cell senescence.

3. The method for delaying senescence of human mesenchymal stem cells according to claim 1, comprises steps of:
S1, establishing a human mesenchymal stem cell senescence;
S2, using lentivirus to infect the human mesenchymal stem cell senescence model so that the human mesenchymal stem cell senescence model overexpresses the SLFN11, the nucleotide sequence of the SLFN11 gene being shown in SEQ ID NO .1.

4. The method for delaying senescence of human mesenchymal stem cells according to claim 3, wherein the human mesenchymal stem cell senescence model comprises a replicative hMSC senescence model and a DNA damage-induced hMSC senescence model.

5. The method for delaying senescence of human mesenchymal stem cells according to claim 4, wherein the replicative hMSC senescence model is obtained by continuously expanding the hMSC in vitro to the 16th generation; the DNA damage-induced hMSC senescence model is obtained by treating the hMSC with camptothecin (CPT) at a final concentration of 2 µM for 2 hours to induce DNA damage to cause senescence.

6. A modified human mesenchymal stem cell, obtained through overexpression of the SLFN11 gene in the human mesenchymal stem cells using the method according to any one of claims 1 to 5, the nucleotide sequence of the SLFN11 gene being shown in SEQ ID NO .1.

7. The modified human mesenchymal stem cell according to claim 6, wherein the overexpression of the SLFN11 gene is achieved by infecting the human mesenchymal stem cells with lentivirus.

8. The modified human mesenchymal stem cell according to claim 7, wherein the lentivirus is an overexpressed virus HBLV-h-SLFN11-3xflag-ZsGreen-PURO.

9. Use of the modified human mesenchymal stem cell according to claim 6 in the preparation of a drug for treating ulcerative colitis.

10. A drug combination, comprising the modified human mesenchymal stem cell according to claim 6.

## Patentansprüche

1. Verfahren zum Verzögern der Seneszenz humaner mesenchymaler Stammzellen, wobei ein SLFN11-Gen in den humanen mesenchymalen Stammzellen überexprimiert wird und die Nukleotidsequenz des SLFN11-Gens in SEQ ID NO. 1 gezeigt ist.

2. Verfahren zum Verzögern der Seneszenz humaner mesenchymaler Stammzellen nach Anspruch 1, wobei die Seneszenz humaner mesenchymaler Stammzellen eine replikative Zellseneszenz und eine durch DNA-Schäden induzierte Zellseneszenz umfasst.

3. Verfahren zum Verzögern der Seneszenz humaner mesenchymaler Stammzellen nach Anspruch 1, umfassend die Schritte: S1, Etablieren einer Seneszenz humaner mesenchymaler Stammzellen; S2, Verwenden von Lentivirus zum Infizieren des Modells der Seneszenz humaner mesenchymaler Stammzellen, so dass das Modell der Seneszenz humaner mesenchymaler Stammzellen das SLFN11 überexprimiert, wobei die Nukleotidsequenz des SLFN11-Gens in SEQ ID NO. 1 gezeigt ist.

4. Verfahren zum Verzögern der Seneszenz humaner mesenchymaler Stammzellen nach Anspruch 3, wobei das Modell der Seneszenz humaner mesenchymaler Stammzellen ein replikatives hMSC-Seneszenzmodell und ein durch DNA-Schäden induziertes hMSC-Seneszenzmodell umfasst.

5. Verfahren zum Verzögern der Seneszenz humaner mesenchymaler Stammzellen nach Anspruch 4, wobei das replikative hMSC-Seneszenzmodell durch kontinuierliches Expandieren der hMSC in vitro bis zur 16. Generation erhalten wird; das durch DNA-Schäden induzierte hMSC-Seneszenzmodell erhalten wird durch Behandeln der hMSC mit Camptothecin (CPT) in einer Endkonzentration von 2 µM für 2 Stunden, um eine DNA-Schädigung zu induzieren, um eine Seneszenz zu verursachen.

6. Modifizierte humane mesenchymale Stammzelle, erhalten durch Überexpression des SLFN11-Gens in den humanen mesenchymalen Stammzellen unter Verwendung des Verfahrens nach einem der Ansprüche 1 bis 5, wobei die Nukleotidsequenz des SLFN11-Gens in SEQ ID NO. 1 gezeigt ist.

7. Modifizierte humane mesenchymale Stammzelle nach Anspruch 6, wobei die Überexpression des SLFN11-Gens durch Infizieren der humanen mesenchymalen Stammzellen mit Lentivirus erreicht wird.

8. Modifizierte humane mesenchymale Stammzelle nach Anspruch 7, wobei das Lentivirus ein überexprimiertes Virus HBLV-h-SLFN11-3xflag-ZsGreen-PURO ist.

9. Verwendung der modifizierten humanen mesenchymalen Stammzelle nach Anspruch 6 bei der Herstellung eines Arzneimittels zur Behandlung von Colitis ulcerosa.

10. Arzneimittelkombination, umfassend die modifizierte humane mesenchymale Stammzelle nach Anspruch 6.

## Revendications

1. Procédé pour retarder la sénescence de cellules souches mésenchymateuses humaines, dans lequel un gène SLFN11 est surexprimé dans les cellules souches mésenchymateuses humaines, et la séquence nucléotidique du gène SLFN11 est présentée dans la SEQ ID NO : 1.

2. Procédé pour retarder la sénescence de cellules souches mésenchymateuses humaines selon la revendication 1, dans lequel la sénescence des cellules souches mésenchymateuses humaines comprend une sénescence cellulaire réplicative et une sénescence cellulaire induite par des dommages à l'ADN.

3. Procédé pour retarder la sénescence de cellules souches mésenchymateuses humaines selon la revendication 1, comprenant les étapes de :
S1, établissement d'une sénescence de cellules souches mésenchymateuses humaines ;
S2, utilisation d'un lentivirus pour infecter le modèle de sénescence de cellules souches mésenchymateuses humaines de sorte que le modèle de sénescence de cellules souches mésenchymateuses humaines surexprime le SLFN11, la séquence nucléotidique du gène SLFN11 étant présentée dans la SEQ ID NO : 1.

4. Procédé pour retarder la sénescence de cellules souches mésenchymateuses humaines selon la revendication 3, dans lequel le modèle de sénescence de cellules souches mésenchymateuses humaines comprend un modèle de sénescence réplicative de hMSC et un modèle de sénescence de hMSC induite par des dommages à l'ADN.

5. Procédé pour retarder la sénescence de cellules souches mésenchymateuses humaines selon la revendication 4, dans lequel le modèle de sénescence réplicative de hMSC est obtenu par expansion continue des hMSC in vitro jusqu'à la 16e génération ; le modèle de sénescence de hMSC induite par des dommages à l'ADN est obtenu par traitement des hMSC avec de la camptothécine (CPT) à une concentration finale de 2 µM pendant 2 heures pour induire des dommages à l'ADN afin de provoquer la sénescence.

6. Cellule souche mésenchymateuse humaine modifiée, obtenue par surexpression du gène SLFN11 dans les cellules souches mésenchymateuses humaines à l'aide du procédé selon l'une quelconque des revendications 1 à 5, la séquence nucléotidique du gène SLFN11 étant présentée dans la SEQ ID NO : 1.

7. Cellule souche mésenchymateuse humaine modifiée selon la revendication 6, dans laquelle la surexpression du gène SLFN11 est réalisée en infectant les cellules souches mésenchymateuses humaines avec un lentivirus.

8. Cellule souche mésenchymateuse humaine modifiée selon la revendication 7, dans laquelle le lentivirus est un virus surexprimé HBLV-h-SLFN11-3xflag-ZsGreen-PURO.

9. Utilisation de la cellule souche mésenchymateuse humaine modifiée selon la revendication 6 dans la préparation d'un médicament pour le traitement de la colite ulcéreuse.

10. Association médicamenteuse, comprenant la cellule souche mésenchymateuse humaine modifiée selon la revendication 6.
